# EUROPÄISCHE PATENTANMELDUNG

(11) **EP 2 106 744 A1**
(43) Veröffentlichungstag der Anmeldung: **07.10.2009**
(21) Anmeldenummer: 09155400.6
(22) Anmeldetag: 17.03.2009
(51) Int. Cl.: A61B 5/00

(54) **Vorrichtung und Verfahren zur Beeinflussung des Schlafs einer Person, Bedieneinheit und Computerprogrammprodukt**

(30) Priorität: 02.04.2008 DE 102008016855
(71) Anmelder: Pfeifer, Herr Thomas, 60323 Frankfurt am Main (DE)
(72) Erfinder: Pfeifer, Herr Thomas, 60323 Frankfurt am Main (DE)
(74) Vertreter: Barth, Stephan Manuel

(57) **Zusammenfassung**

Die Erfindung schafft eine Vorrichtung und ein Verfahren zur Beeinflussung des Schlafs einer Person. Die Vorrichtung umfasst eine Sensoreinrichtung zum Erfassen einer aktuellen Drehposition des Körpers der Person um die Körperlängsachse, eine Aktoreinrichtung zum Zuführen von einem vorgebbaren Reizmuster an den Körper, und eine Bedieneinheit mit eine Auswahleinrichtung zum Auswählen mindestens einer erwünschten und/oder mindestens einer verbotenen Drehposition des Körpers der Person um die Körperlängsachse, einer Vergleichseinrichtung zum Vergleichen der aktuellen Drehposition und der mindestens einen erwünschten und/oder mindestens einen verbotenen Drehposition und Ausgeben eines entsprechenden Lagesignals, und einer Steuereinrichtung, welche einrichtbar ist, ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

## Beschreibung

Die vorliegende Erfindung betrifft eine Vorrichtung und ein Verfahren zur Beeinflussung des Schlafs einer Person, eine Bedieneinheit und ein Computerprogrammprodukt.

Aus der DE 101 14 205 B1 ist eine Vorrichtung zur Korrektur der Schlafhaltung einer Person mit einer Befestigungseinrichtung, die als Bauchgürtel an den Körpers der Person anbringbar ist, und mit zumindest einer an der Befestigungseinrichtung angebrachten Sensoreinrichtung zum Erfassen einer Bauchlage bzw. eines Bereichs um die Bauchlage als vorbestimmte Drehposition des Körpers der Person bekannt. Eine an der Befestigungseinrichtung angebrachte Aktoreinrichtung leitet einen vorgebbaren Hautreiz im Bereich der Befestigungseinrichtung an den Körper der Person ansprechend auf eine Erfassung der Sensoreinrichtung, dass die Drehposition die Bauchlage bzw. den Bereichs um die Bauchlage einnimmt.

Fig. 7 ist ein Blockdiagramm einer aus der DE 101 14 205 B1 bekannten Vorrichtung zur Korrektur der Schlafhaltung einer Person.

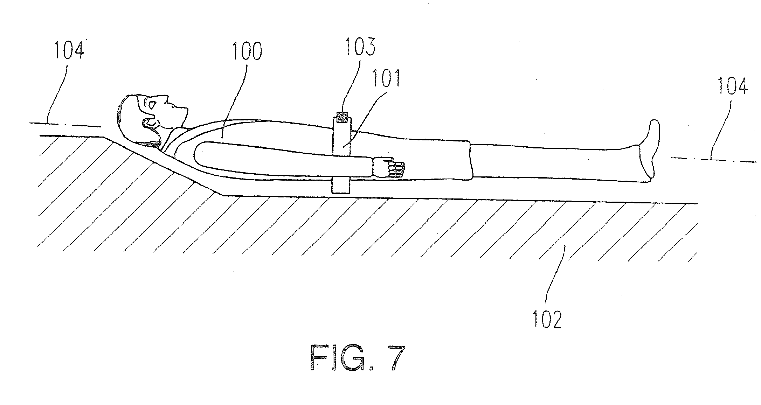

In der in Fig. 7 gezeigten Prinzipskizze ist ein Körper 100 eines Menschen während des Schlafens auf einer Unterlage 102 in einer vorzugsweise horizontalen Position angeordnet, d.h. eine Körperlängsachse 104 ist parallel zu der horizontal ausgerichteten Unterlage 102 ausgerichtet, wobei eine anfängliche Schlafposition eine Rückenlage ist. Mittels einer Befestigungseinrichtung 101, die beispielsweise als ein Bauch-Gürtel ausgebildet sein kann, ist ein Sensor-Aktor-Modul 103 an dem Körper 100 des Menschen angebracht.

Die Befestigungseinrichtung 101 kann Bewegungen des Körpers 100 widerstehen. Weiterhin ist die Anbringung der Befestigungseinrichtung 101 an dem Körper 100 des Menschen für den Schlaf des Menschen nur geringfügig störend, da prinzipiell Bewegungen des Körpers 100 in sämtliche Richtungen, insbesondere eine Drehbewegung des Körpers 100 eines Menschen um seine Körperlängsachse 104 ermöglicht werden.

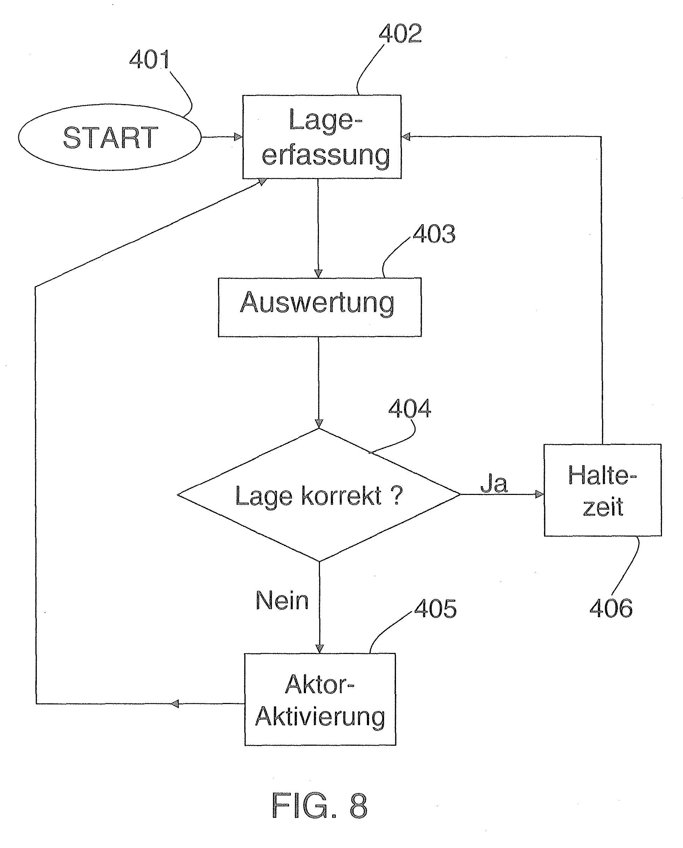

Fig. 8 ist ein Flußdiagramm, welches den Ablauf eines Verfahrens zur Korrektur der Schlafhaltung einer Person unter Verwendung der bekannten Vorrichtung nach Fig. 7 veranschaulicht.

In einem Schritt 401 wird nach einer Inbetriebnahme der erfindungsgemäßen Vorrichtung zunächst eine Positionserfassung in einem Schritt 402 durchgeführt. Die Ausgangssignale von der Sensoreinrichtung werden in einem Schritt 403 hinsichtlich einer Position, insbesondere einer Drehposition um eine Längsachse des Körpers 100 ausgewertet.

In einem Schritt 404 wird bestimmt, ob eine bezüglich einer vorgegebenen Sollposition festgestellte Position korrekt ist oder nicht (korrekt = Rückenlage, seitliche Lage, ...). Wird in dem Schritt 404 bestimmt, daß die Position korrekt ist, so wird in einem Schritt 406 eine vorgebbare Zeit, eine sogenannte Haltezeit abgewartet, bis die Verarbeitung zu dem Schritt 402 zurückkehrt.

Anschließend werden die Schritte 402, 403 und 404 erneut durchlaufen. Wird in dem Schritt 404 bestimmt, daß die Position nicht korrekt ist (nicht korrekt = Bauchlage), dann wird in einem Schritt 405 eine Aktivierung der Aktoreinrichtung eingeleitet. Eine Reizausgabe der Aktoreinrichtungen führt zu einer Reizung des Körpers 100 eines Menschen mittels Piezoaktor, Heizelement, Kühlelement, Vibration, etc., wie oben beschrieben, so daß der schlafende Mensch aufgrund dieser Reizung seine Schlafposition wechseln wird. Nach einer Aktor-Aktivierung in dem Schritt 405, die in ihrer Zeitdauer und in ihrer Intensität durch die Verarbeitungseinheit vorgebbar ist, kehrt die Verarbeitung zu dem Schritt 402 zurück, wo eine erneute Positionserfassung durchgeführt wird. Die Verarbeitung durchläuft wiederum die Schritte 402, 403 und 404, bis in dem Schritt 404 bestimmt wird, daß die eingenommene Position korrekt ist.

Wird in dem Schritt 404 bestimmt, daß die Position noch immer nicht korrekt ist, schreitet die Verarbeitung erneut zu dem Schritt 405 und anschließend zu dem Schritt 402 fort.

Andernfalls schreitet, wenn in dem Schritt 404 bestimmt wird, daß die Position korrekt ist, die Verarbeitung zu dem Schritt 406 und, nach einer Haltezeit, zu dem Schritt 402 fort. Eine Haltezeit, die in dem Schritt 406 eingestellt wird, wird so gewählt, daß eine Positionserfassung nur zu bestimmten, durch die Verarbeitungseinheit 304 vorgebbaren Zeitpunkten ausgeführt wird, so daß eine allzu häufige Reizung des Körpers 100 eines Menschen und damit eine Gefahr eines Aufwachens vermieden wird.

Weiterhin dient eine vorgebbare Haltezeit dazu, den Energiebedarf der Gesamtanordnung zu reduzieren und eine Belastung der Energieversorgungseinheit 305 gering zu halten.

Durch diese bekannte Vorrichtung lassen sich ein unerwünschter Druck und/oder unerwünschten Zugspannungen, welche zu Blockierungen im Bereich der Wirbelgelenke führen, insbesondere der Halswirbelgelenke, was bekannt ist unter dem Namen "Torticollis" (= akuter Schiefhals), vermeiden.

Aus der DE 83 28 640 U1 ist eine Vorrichtung zur Korrektur der Schlafhaltung einer Person bekannt, bei der ein im Ohr des Benutzers tragbarer Ohrknopf mit einem durch Lageveränderung auslösbaren Signalerzeuger versehen ist.

Es ist eine Aufgabe der vorliegenden Erfindung, eine Vorrichtung und ein Verfahren zur Beeinflussung des Schlafs einer Person, eine Bedieneinheit und ein Computerprogrammprodukt zu schaffen, welche flexibler einsetzbar sind.

Die Erfindung schafft eine Vorrichtung zur Beeinflussung des Schlafs einer Person nach Anspruch 1 und ein Verfahren zur Beeinflussung des Schlafs einer Person nach Anspruch 16, eine Bedieneinheit nach Anspruch 17 und ein Computerprogrammprodukt nach Anspruch 18.

Die erfindungsgemäßen Gegenstände weisen zahlreiche Vorteile auf. Insbesondere ermöglichen Sie einen flexiblen Einsatz in unterschiedlichen Therapien.

In den Unteransprüchen finden sich vorteilhafte Weiterbildungen und Verbesserungen des jeweiligen Gegenstandes der Erfindung.

Gemäß einer bevorzugten Weiterbildung ist eine zweite Sensoreinrichtung zum Erfassen von Schnarchen und zum Ausgeben eines entsprechenden Schnarchsignals vorgesehen, wobei die Steuereinrichtung durch eine zweite Auswahleinrichtung einrichtbar ist, welche in der Bedieneinheit vorgesehen ist,
ansprechend auf das Schnarchsignal die Aktoreinrichtung zum Zuführen von dem vorgebbaren Reizmuster an den Körper anzusteuern, wenn das Schnarchsignal anzeigt, dass die Person schnarcht,
und/oder
ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

Gemäß einer weiteren bevorzugten Weiterbildung ist eine dritte Sensoreinrichtung zum Erfassen einer Kreislaufkenngrösse der Person, und zum Ausgeben eines entsprechenden Kreislaufsignals vorgesehen, wobei die Steuereinrichtung durch die zweite Auswahleinrichtung einrichtbar ist,
ansprechend auf das Kreislaufsignal die Aktoreinrichtung zum Zuführen von dem vorgebbaren Reizmuster an den Körper anzusteuern, wenn das Kreislaufsignal anzeigt, dass der Kreislauf der Person gestört ist,
und/oder
ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht,
und/oder
ansprechend auf das Schnarchsignal die Aktoreinrichtung zum Zuführen von dem vorgebbaren Reizmuster an den Körper anzusteuern, wenn das Schnarchsignal anzeigt, dass die Person schnarcht.

Gemäß einer weiteren bevorzugten Weiterbildung ist eine dritte Auswahleinrichtung zum Auswählen des Reizmusters vorhanden, welche in der Bedieneinheit vorgesehen ist.

Gemäß einer weiteren bevorzugten Weiterbildung ist die dritte Auswahleinrichtung derart gestaltet, dass das Reizmuster unterschiedlich wählbar ist entsprechend dem Vorliegen verschiedener Kombinationen von Lagesignal, Schnarchsignal und Kreislaufsignal, welche ein Ansteuern der Aktoreinrichtung durch die Steuereinrichtung zur Folge haben.

Gemäß einer weiteren bevorzugten Weiterbildung weist die zweite Sensoreinrichtung ein Mikrofon auf. Vorzugsweise ist die Bandbreite des Mikrofons schnarchspezifisch ist und vorzugsweise zwischen 30 und 1600 Hz.

Gemäß einer weiteren bevorzugten Weiterbildung ist die Empfindlichkeit und/oder Frequenzcharakteristik der zweiten Sensoreinrichtung einstellbar.

Gemäß einer weiteren bevorzugten Weiterbildung weist die dritte Sensoreinrichtung einen Herzfrequenzsensor und/oder einen Blutsauerstoffsättigungssensor auf.

Gemäß einer weiteren bevorzugten Weiterbildung sind die Herzfrequenz und/oder die Blutsauerstoffsättigung, welche anzeigen, dass der Kreislauf der Person gestört ist, einstellbar.

Gemäß einer weiteren bevorzugten Weiterbildung ist ein Haltezeitglied vorgesehen, das ausgelegt ist, einen Betrieb der Vorrichtung vorgebbar zu unterbrechen.

### ZEICHNUNGEN

Ausführungsbeispiele der Erfindung sind in den Zeichnungen dargestellt und in der nachfolgenden Beschreibung näher erläutert.

In den Zeichnungen zeigen:
- Fig. 1: ein Blockdiagramm einer ersten Ausführungsform der erfindungsgemäßen Vorrichtung zur Beeinflussung des Schlafs einer Person;
- Fig. 2: ein Flußdiagramm, welches den Ablauf eines Verfahrens zur Beeinflussung des Schlafs einer Person unter Verwendung der Vorrichtung gemäß der ersten Ausführungsform veranschaulicht;
- Fig. 3: ein Blockdiagramm einer zweiten Ausführungsform der erfindungsgemäßen Vorrichtung zur Beeinflussung des Schlafs einer Person;
- Fig. 4: ein Flußdiagramm, welches den Ablauf eines Verfahrens zur Beeinflussung des Schlafs einer Person unter Verwendung der Vorrichtung gemäß der zweiten Ausführungsform veranschaulicht;
- Fig. 5: ein Blockdiagramm einer dritten Ausführungsform der erfindungsgemäßen Vorrichtung zur Beeinflussung des Schlafs einer Person;
- Fig. 6: ein Flußdiagramm, welches den Ablauf eines Verfahrens zur Beeinflussung des Schlafs einer Person unter Verwendung der Vorrichtung gemäss der dritten Ausführungsform veranschaulicht;
- Fig. 7: ein Blockdiagramm einer aus der DE 101 14 205 B1 bekannten Vorrichtung zur Korrektur der Schlafhaltung einer Person; und
- Fig. 8: ein Flußdiagramm, welches den Ablauf eines Verfahrens zur Korrektur der Schlafhaltung einer Person unter Verwendung der bekannten Vorrichtung nach Fig. 7 veranschaulicht.

In den Figuren bezeichnen gleiche Bezugszeichen gleiche oder funktionsgleiche Komponenten.

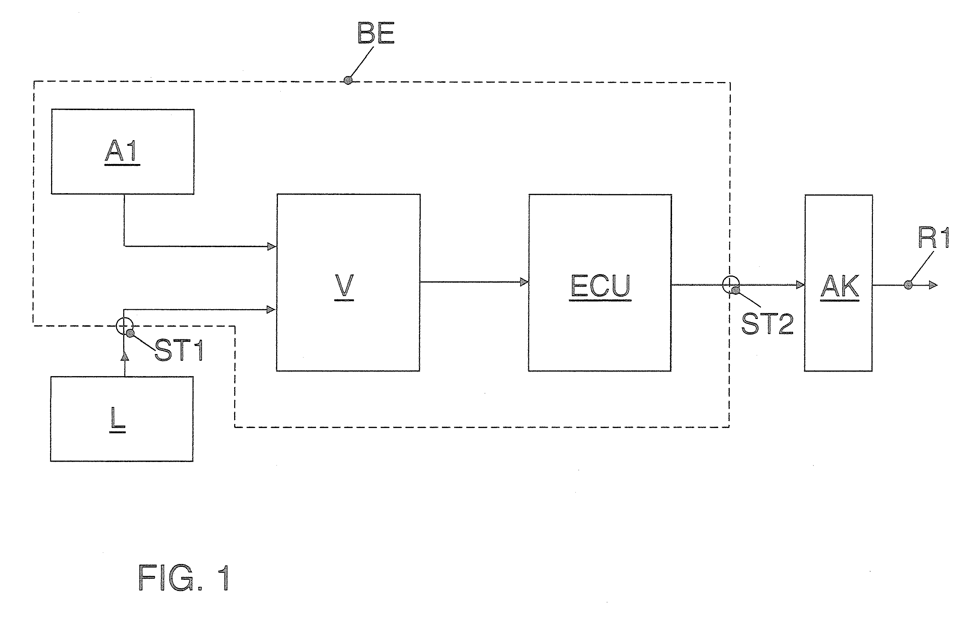

Fig. 1 ist ein Blockdiagramm einer ersten Ausführungsform der erfindungsgemäßen Vorrichtung zur Beeinflussung des Schlafs einer Person.

In Fig. 1 bezeichnet Bezugszeichen BE eine Bedieneinheit für eine Vorrichtung zur Beeinflussung des Schlafs einer Person, bespielsweise ein Standalone-Gerät oder ein an einer Person befestigbares Gerät. Die Bedieneinheit BE weist einen ersten Anschluss ST1 zum Anschließen einer an einer Person anbringbaren Sensoreinrichtung L zum Erfassen einer aktuellen Drehposition des Körpers der Person um die Körperlängsachse auf. Weiterhin weist die Bedieneinheit BE einen zweiten Anschluss ST2 zum Anschließen einer Aktoreinrichtung AK zum Zuführen von einem vorgegebenen Reiz an den Körper auf, beispielsweise einen Vibrationsreiz.

Eine entsprechende Sensoreinrichtung L ist an den ersten Anschluss ST1 angeschlossen, und eine entsprechende Aktoreinrichtung AK ist an den zweiten Anschluss ST2 angeschlossen, welche beim vorliegenden Beispiel beide in einem Bauch-Gürtel ausgebildet sind, also als Sensor-Aktor-Modul 103 an dem Körper 100 des Menschen angebracht sind (vgl. Fig. 7).

Untergebracht in der Bedieneinheit BE sind eine erste Auswahleinrichtung A1 zum Auswählen mindestens einer erwünschten und/oder mindestens einer verbotenen Drehposition des Körpers der Person um die Körperlängsachse. Eine derartige erste Auswahleinrichtung A1 kann beispielsweise in Form eines Displays mit einer zugehörigen Set-Point-Einstellungseinrichtung vorliegen. Auch kann es sich bei einfachen Ausführungen um einen einfachen Drehknopf bzw. mehrere Drehknöpfe bei Einstellung eines Drehpositionsbereichs handeln.

Einer in der Bedieneinheit BE befindlichen Vergleichseinrichtung V werden die vorgegebenen erwünschen und/oder verbotenen Drehpositionen eingegeben sowie die aktuelle Drehposition, welche von der ersten Sensoreinrichtung L erfasst wird.

Die Vergleichseinrichtung V vergleicht die aktuelle Drehposition und die erwünschten und/oder verbotenen Drehposition und gibt ein entsprechendes Lagesignal an eine ebenfalls in der Bedieneinheit BE befindliche Steuereinrichtung ECU aus.

Die Steuereinrichtung ECU steuert ansprechend auf das Lagesignal die Aktoreinrichtung AK zum Zuführen von dem vorgebbaren Reizmuster R1 an den Körper an, wenn das Lagesignal anzeigt, dass die aktuelle Drehposition nicht den erwünschten Drehpositionen entspricht und/oder den verbotenen Drehpositionen entspricht.

Beispielsweise kann es sich bei der ersten Sensoreinrichtung L um einen optischen Sensor handeln, der auf der Basis des Sagnac-Effektes arbeitet, in dem eine Faserkreiseleinrichtung bereitgestellt ist, mit der Drehungen des Körpers um die Längsachse erfassbar sind.

Mit der Vorrichtung gemäß der ersten Ausführungsform kann also eine beliebige Drehposition des Körpers der Person um die Körperlängsachse entweder als erwünscht oder als verboten festgelegt werden. Somit können im praktischen Einsatz im Gegensatz zum Stand der Technik nicht nur Bauchlagen verhindert werden, sondern insbesondere auch Rückenlagen, welche für Schnarch-Patienten gefährlich sein können. Auch können bei bestimmten Verletzungen Seitenlagen zu vermeiden sein.

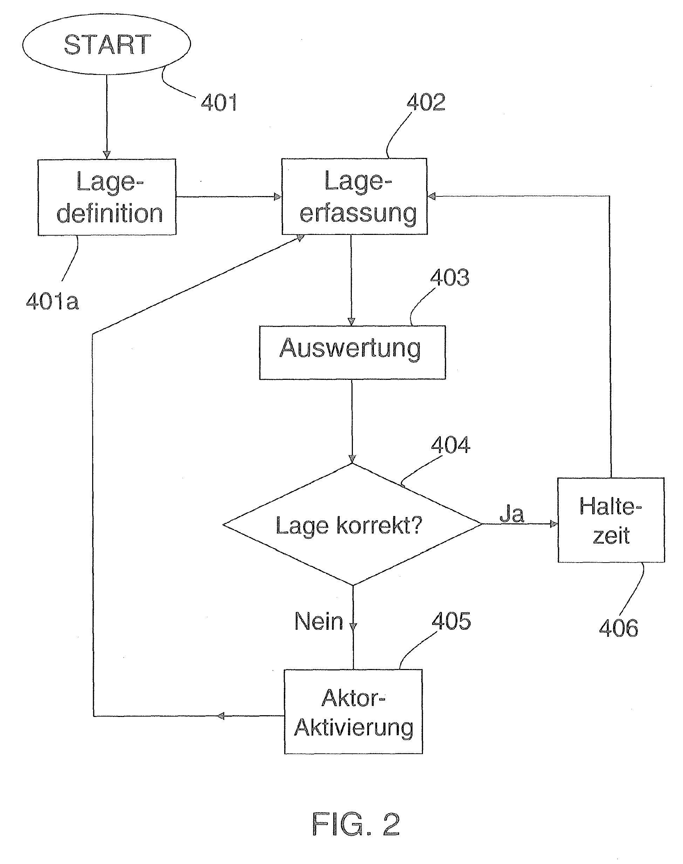

Fig. 2 ist ein Flußdiagramm, welches den Ablauf eines Verfahrens zur Beeinflussung des Schlafs einer Person unter Verwendung der Vorrichtung gemäß der ersten Ausführungsform veranschaulicht.

Im Unterschied zu dem oben in Zusammenhang mit Fig. 8 erläuterten bekannten Verfahren umfasst das Verfahren gemäß Fig. 2 einen zusätzlichen Schritt 401a, welcher als Lagedefinition bezeichnet ist. Dieser Schritt 401a wird unmittelbar nach dem Start 401 durchgeführt, und zwar vor dem Beginn der Lageerfassung im Schritt 402.

Im Schritt 401a gibt der Benutzer die erwünschten und/oder die verbotenen Drehpositionen bzw. Lagen sowie optionellerweise auch dazugehörige Toleranzbereiche mittels der ersten Auswahleinrichtung A1 ein.

Die sich daran anschließende Lageerfassung im Schritt 402 sowie die weiteren Schritte 403, 404, 405, 406 erfolgen derart, wie bereits im Zusammenhang mit Fig. 8 ausführlich erläutert.

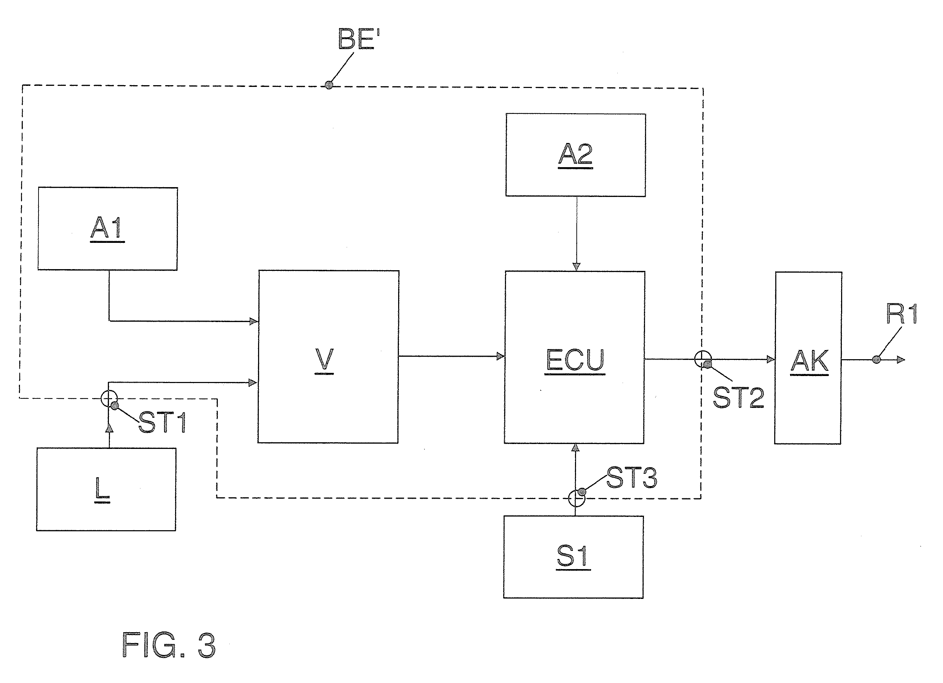

Fig. 3 ist ein Blockdiagramm einer zweiten Ausführungsform der erfindungsgemäßen Vorrichtung zur Beeinflussung des Schlafs einer Person.

Bei der zweiten Ausführungsform gemäß Fig. 3 bezeichnet Bezugszeichen BE' eine Bedieneinheit, welche im Vergleich zur Bedieneinheit BE gemäß der ersten Ausführungsform erweitert ist.

Insbesondere umfasst die Bedieneinheit BE' einen dritten Anschluss ST3 zum Anschließen einer zweiten Sensoreinrichtung S1 zum Erfassen von Schnarchen und zum Ausgeben eines entsprechenden Schnarchsignals an die Steuereinrichtung ECU. Die zweite Sensoreinrichtung S1 kann beispielsweise ein Mikrofon aufweisen, dessen Bandbreite vorzugsweise schnarchspezifisch ist und beispielsweise zwischen 30 und 1600 Hz liegt. Optionellerweise kann die Empfindlichkeit und/oder die Frequenzcharakteristik dieses Mikrofons einstellbar sein, um eine Feineinstellung entsprechend dem jeweiligen Schnarchpatienten zu ermöglichen.

Des Weiteren weist die Bedieneinrichtung BE' zusätzlich eine zweite Auswahleinrichtung A2 auf, wodurch die Steuereinrichtung ECU einrichtbar ist, entweder nur auf das Ausgangssignal der Vergleichseinrichtung V zu reagieren oder nur auf das Schnarchsignal der zweiten Sensoreinrichtung S1 in Form des Mikrofons zu reagieren oder nur dann zu reagieren, falls sowohl das Ausgangssignal der Vergleichseinrichtung V als auch das Schnarchsignal einen Eingriff der Aktoreinrichtung AK erfordern.

Mit anderen Worten lässt sich durch die zweite Auswahleinrichtung A2 festlegen, ob ein Aktoreingriff bereits aufgrund der Tatsache, dass die Drehposition falsch ist, erfolgen soll oder bereits aufgrund der Tatsache, dass ein Schnarchen vorliegt, oder nur dann, falls ein Schnarchen in einer unerwünschten Lage auftritt, also beide Eingriffsbedingungen erfüllt sein müssen. Beim Schnarchpatienten ist die Rückenlage unerwünscht, in der der Unterkiefer und die Zunge schwerkraftbedingt zurückfallen könnten, was zu einer Einengung der Luftwege führen könnte bzw. zu einem vollständigen Kollaps der obstruktiven Schlafapnoe, was 10% der Schnarcher betrifft.

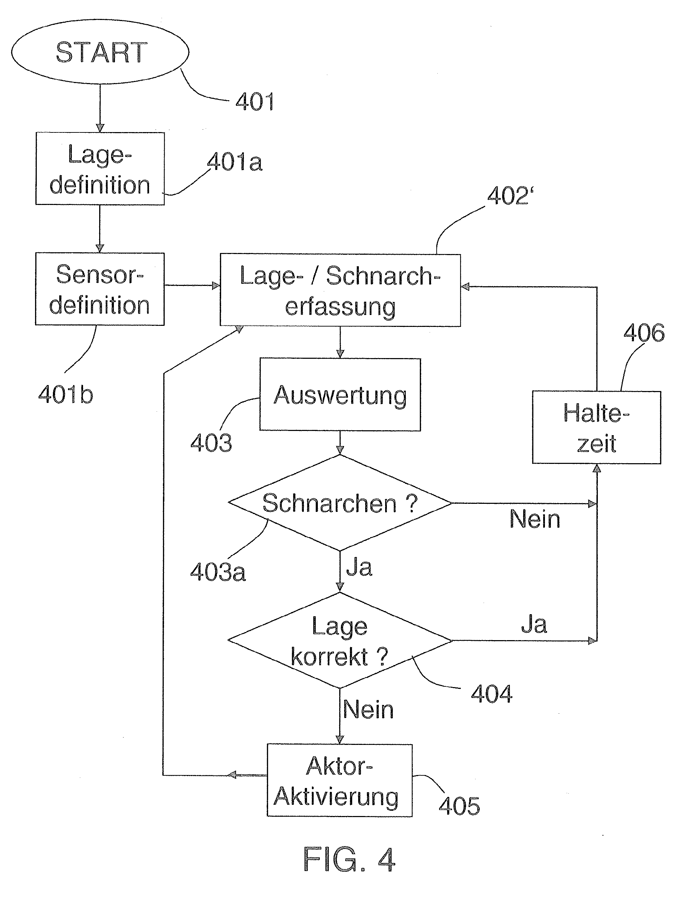

Fig. 4 ist ein Flußdiagramm, welches den Ablauf eines Verfahrens zur Beeinflussung des Schlafs einer Person unter Verwendung der Vorrichtung gemäß der zweiten Ausführungsform veranschaulicht.

Bei dem in Fig. 4 dargestellten Verfahren gibt es zusätzlich zu dem in Zusammenhang mit Fig. 2 beschriebenen Verfahren einen Schritt 401b, in dem mittels der zweiten Auswahleinrichtung A2 eine Sensordefinition vorgenommen wird, wie zuvor erläutert. Mit der Auswahleinrichtung A2, welche beispielsweise in Form eines Drehschalters oder eines sonstigen Schalters vorliegt, können also die erste Sensoreinrichtung L oder die zweite Sensoreinrichtung S1 oder die erste und die zweite Sensoreinrichtung L, S1 als maßgeblich für die Festlegung eines Eingriffs der Aktoreinrichtung AK durch die Steuereinrichtung ECU festgelegt werden.

Sind, wie im vorliegenden Ausführungsbeispiel, sowohl die erste Sensoreinrichtung L als auch die zweite Sensoreinrichtung S1 als maßgeblich für einen Eingriff ausgewählt worden, so findet im Schritt 402' eine Lageerfassung und Schnarcherfassung statt. Nach dem Auswerteschritt 403 findet ein Schritt 403a statt, in dem bestimmt wird, ob die zweite Sensoreinrichtung S1 anzeigt, dass ein Schnarchen vorliegt. Ist dies nicht der Fall, verzweigt das Programm zum Schritt 406, in dem das Haltezeitglied durchlaufen wird, und dann erneut zu Schritt 402' zur Lageerfassung und Schnarcherfassung.

Lieg ein Schnarchen im Schritt S403a vor, so läuft das Programm weiter zum Schritt 404, in dem überprüft wird, ob die Lage korrekt ist, und zwar entsprechend der im Schritt 401a eingestellten Lagedefinitionen. Ist die Lage korrekt, so verzweigt das Programm ebenfalls zum Schritt 406, in dem das Haltezeitglied durchlaufen wird, und dann erneut zu Schritt 402' zur Lageerfassung und Schnarcherfassung.

Falls beide Bedingungen erfüllt sind, also sowohl ein Schnarchen in Schritt 403a erfasst wurde als auch eine inkorrekte Lage in Schritt 404 erfasst wurde, dann kommt es im Schritt 405 zu einer Aktor-Aktivierung, beispielsweise in Form eines Auslösens der Vibration der Vibrationseinrichtung entsprechend dem Reizmuster R1, um den Patienten zu bewegen, um eine andere Lage, beispielsweise die Bauchlage zu drehen.

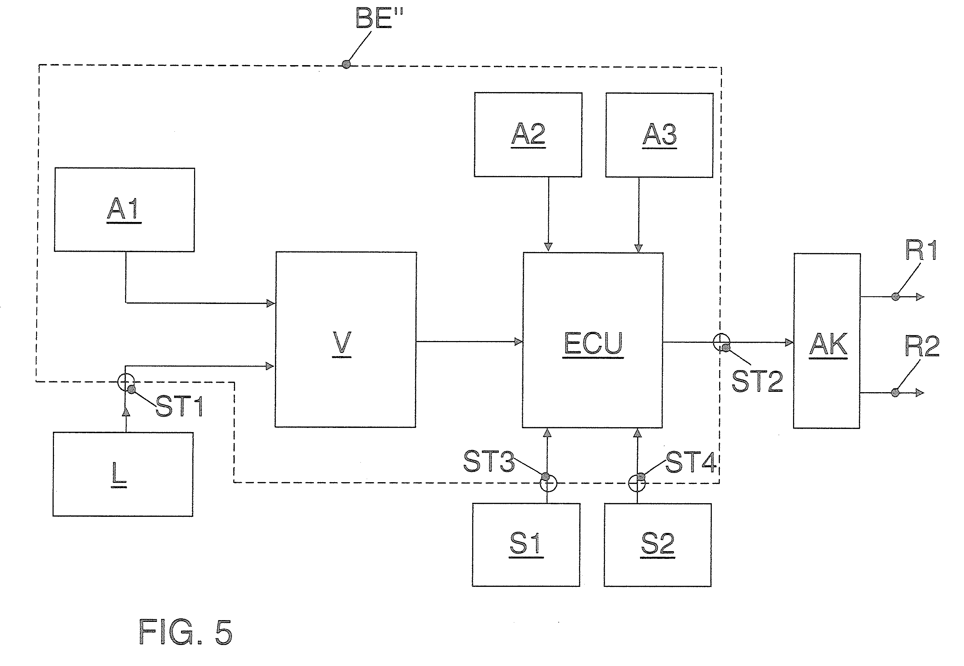

Fig. 5 ist ein Blockdiagramm einer dritten Ausführungsform der erfindungsgemäßen Vorrichtung zur Beeinflussung des Schlafs einer Person.

Bei der dritten Ausführungsform gemäß Fig. 5 ist die Bedieneinheit mit dem Bezugszeichen BE'' bezeichnet. Die Bedieneinheit BE'' weist neben den Komponenten der Bedieneinheit BE' einen vierten Anschluss ST4 zum Anschließen einer dritten Sensoreinrichtung S2 auf, wobei die dritte Sensoreinchrichtung S2 beim vorliegenden Beispiel ein Blutsauerstoffsättigungssensor ist, der die Blutsauerstoffkonzentration im Blut des Patienten misst und ein entsprechendes Blutsauerstoffsignal als Kreislaufsignal ausgibt.

Die Steuereinrichtung ECU empfängt das Blutsauerstoffsignal der zweiten Sensoreinrichtung S2 und kann durch die zweite Auswahleinrichtung A2 eingerichtet werden, ansprechend auf das Blutsauerstoffsignal die Aktoreinrichtung AK zum Zuführen von einem vorgebbaren Reizmuster anzusteuern, wenn das Blutsauerstoffsignal anzeigt, dass der Kreislauf der Person gestört ist.

Beispielsweise kann ein Abfall der Sauerstoffsättigung um 4% eine kritische Situation für den Patienten darstellen, welcher einen derartigen Eingriff rechtfertigt. Optionellerweise ist ein derart kritischer Wert der Sauerstoffsättigung voreinstellbar bzw. ein entsprechender Grenzwert in der zweiten Sensoreinrichtung S2 einstellbar.

Wie bei der zweiten Ausführungsform kann durch die zweite Auswahleinrichtung A2 festgelegt werden, ob die Steuereinrichtung ECU nur auf das Lagesignal, nur auf das Schnarchsignal, nur auf das Blutsauerstoffsignal oder beliebige Kombinationen davon reagiert, also einen Aktoreingriff veranlasst.

Weiterhin vorgesehen in der Bedieneinheit BE2' ist eine dritte Auswahleinrichtung A3, mit der festlegbar ist, welches Reizmuster R1, R2 entsprechend dem Vorliegen verschiedener Kombination von Lagesignal, Schnarchsignal und Blutsauerstoffsignal veranlasst wird. Beispielsweise ist bei dem vorliegenden Beispiel festgelegt, dass bei dem Vorliegen von Schnarchen und inkorrekter Lage, aber korrektem Blutsauerstoffsignal ein Reiz R1 ausgegeben wird, beispielsweise die besagte Vibration.

Liegt hingegen zusätzlich zum Schnarchen und einer inkorrekten Lage eine zu niedrige Blutsauerstoffkonzentration vor, so kann durch die Auswahleinrichtung 3 im Voraus eingestellt werden, dass ein zweites Reizmuster R2 ausgegeben wird, welches beispielsweise einen stärkeren Eingriff auf den Patienten ausübt, beispielsweise ein Wecksignal einer akustischen Weckeinrichtung oder ein stärkerer Vibrationsreiz, wobei das stärkere Aktorsignal gewährleisten soll, dass der Patient in jedem Fall aufwacht bzw. seine Lage wechselt.

Auch die dritte Auswahleinrichtung A3 kann in Form eines Drehschalters oder sonstigen Schalters vorliegen. Die Aktoreinrichtung AK weiß anhand des von der Steuereinrichtung ECU über den zweiten Eingang ST2 ausgegebenen Signals, welchen speziellen Aktor sie aktivieren soll, beispielsweise Vibrator für das Reizmuster R1 und/oder Wecker für das Reizmuster R2.

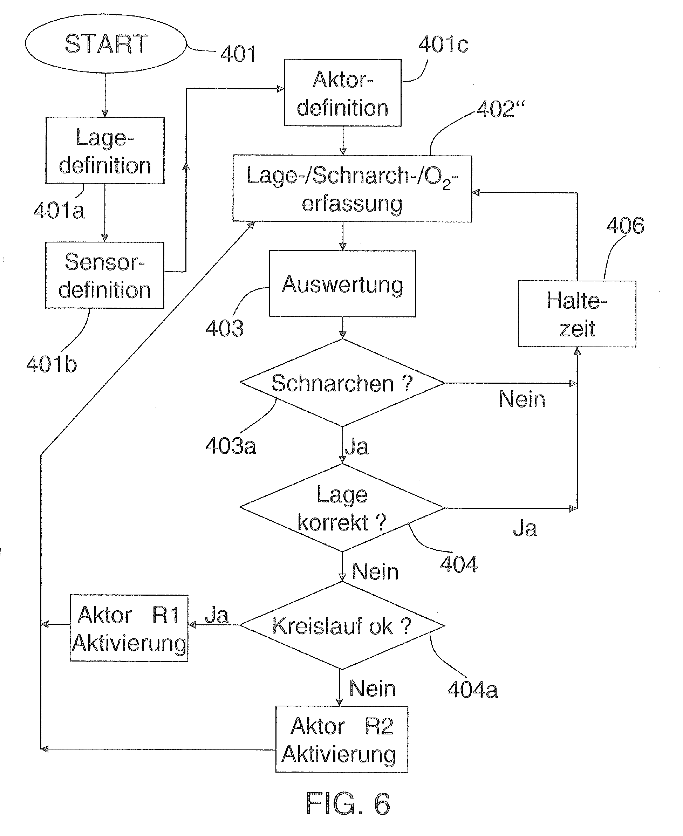

Fig. 6 ist ein Flußdiagramm, welches den Ablauf eines Verfahrens zur Beeinflussung des Schlafs einer Person unter Verwendung der Vorrichtung gemäss der dritten Ausführungsform veranschaulicht.

Bei dem Verfahren gemäß Fig. 6 gibt es zusätzlich zu dem Verfahren gemäß Fig. 4 den Schritt 401c, in dem vor einer Lage-/Schnarch/O₂-Erfassung im Schritt 402'' eine Aktordefinition durchgeführt wird, in der bestimmt wird, bei welchen Sensorkriterien welche Aktoreinrichtung tätig werden soll. Bei diesem Beispiel also, wird definiert, dass falsche Lage und zusätzlich Schnarchen das Reizmuster R1 ausgelöst wird und, falls zusätzlich der Kreislauf gestört ist entsprechend dem Blutsauerstoffsignal, das Reizmuster R2 ausgelöst wird.

Weiterhin wird, falls die Schritte 403a und 404 mit JA beantwortet wurden, nach dem Schritt 404 der Schritt 404a durchgeführt, in dem bestimmt wird, ob der Blutsauerstoffwert in Ordnung ist.

Ist dies der Fall, so wird die Aktoreinrichtung AK zur Ausgabe des Reizmusters R1 angeregt, was im vorliegenden Fall eine Vibrationsauslösung bedeutet.

Wird im Schritt 404a bestimmt, dass der Kreislauf nicht in Ordnung ist, als eine zu niedrige Blutsauerstoffsättigung vorliegt, so wird die Aktoreinrichtung AK durch die Steuereinrichtung ECU veranlasst, das Reizmuster R2 auszugeben, was im vorliegenden Fall ein lautes Wecksignal ist, das ein Aufwachen des Patienten gewährleisten soll.

Obwohl die vorliegende Erfindung vorstehend anhand bevorzugter Ausführungsbeispiele beschrieben wurde, ist sie darauf nicht beschränkt, sondern auf vielfältige Weise modifizierbar.

Insbesondere ist die Erfindung nicht auf die oben beschriebenen Sensor-/Aktor-Kombinationen beschränkt, sondern auf beliebige Sensor-/Aktor-Kombinationen anwendbar.

## Patentansprüche

1. Vorrichtung zur Beeinflussung des Schlafs einer Person mit:
a) einer an der Person anbringbaren ersten Sensoreinrichtung zum Erfassen einer aktuellen Drehposition des Körpers der Person um die Körperlängsachse;
b) einer Aktoreinrichtung zum Zuführen von einem vorgebbaren Reizmuster an den Körper; und
c) einer Bedieneinheit mit
c1) einer ersten Auswahleinrichtung zum Auswählen mindestens einer erwünschten und/oder mindestens einer verbotenen Drehposition des Körpers der Person um die Körperlängsachse;
c2) einer Vergleichseinrichtung zum Vergleichen der aktuellen Drehposition und der mindestens einen erwünschten und/oder mindestens einen verbotenen Drehposition und Ausgeben eines entsprechenden Lagesignals; und
c3) einer Steuereinrichtung, welche einrichtbar ist, ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

2. Vorrichtung zur Beeinflussung des Schlafs einer Person nach Anspruch 1, **gekennzeichnet durch** eine zweite Sensoreinrichtung zum Erfassen von Schnarchen und zum Ausgeben eines entsprechenden Schnarchsignals, wobei die Steuereinrichtung **durch** eine zweite Auswahleinrichtung einrichtbar ist, welche in der Bedieneinheit vorgesehen ist,
ansprechend auf das Schnarchsignal die Aktoreinrichtung zum Zuführen von dem vorgebbaren Reizmuster an den Körper anzusteuern, wenn das Schnarchsignal anzeigt, dass die Person schnarcht,
und/oder
ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

3. Vorrichtung zur Beeinflussung des Schlafs einer Person nach Anspruch 1, **gekennzeichnet durch** eine dritte Sensoreinrichtung zum Erfassen einer Kreislaufkenngrösse der Person, und zum Ausgeben eines entsprechenden Kreislaufsignals, wobei die Steuereinrichtung **durch** die zweite Auswahleinrichtung einrichtbar ist,
ansprechend auf das Kreislaufsignal die Aktoreinrichtung zum Zuführen von dem vorgebbaren Reizmuster an den Körper anzusteuern, wenn das Kreislaufsignal anzeigt, dass der Kreislauf der Person gestört ist,
und/oder
ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht,
und/oder
ansprechend auf das Schnarchsignal die Aktoreinrichtung zum Zuführen von dem vorgebbaren Reizmuster an den Körper anzusteuern, wenn das Schnarchsignal anzeigt, dass die Person schnarcht.

4. Vorrichtung zur Beeinflussung des Schlafs einer Person nach Anspruch 2, **gekennzeichnet durch** eine dritte Sensoreinrichtung zum Erfassen einer Kreislaufkenngrösse der Person, und zum Ausgeben eines entsprechenden Kreislaufsignals, wobei die Steuereinrichtung **durch** die zweite Auswahleinrichtung einrichtbar ist,
ansprechend auf das Kreislaufsignal die Aktoreinrichtung zum Zuführen von dem vorgebbaren Reizmuster an den Körper anzusteuern, wenn das Kreislaufsignal anzeigt, dass der Kreislauf der Person gestört ist,
und/oder
ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

5. Vorrichtung zur Beeinflussung des Schlafs einer Person nach einem der vorhergehenden Ansprüche, **gekennzeichnet durch** eine dritte Auswahleinrichtung zum Auswählen des Reizmusters, welche in der Bedieneinheit vorgesehen ist.

6. Vorrichtung zur Beeinflussung des Schlafs einer Person nach Anspruch 5, wobei die dritte Auswahleinrichtung derart gestaltet ist, dass das Reizmuster unterschiedlich wählbar ist entsprechend dem Vorliegen verschiedener Kombinationen von Lagesignal, Schnarchsignal und Kreislaufsignal, welche ein Ansteuern der Aktoreinrichtung durch die Steuereinrichtung zur Folge haben.

7. Vorrichtung zur Beeinflussung des Schlafs einer Person nach einem der vorhergehenden Ansprüche, **gekennzeichnet durch** eine Befestigungseinrichtung, die als Bauchgürtel an den Körpers der Person anbringbar ist, woran die erste Sensoreinrichtung und die Aktoreinrichtung befestigbar sind.

8. Vorrichtung zur Beeinflussung des Schlafs einer Person nach einem der vorhergehenden Ansprüche, wobei die Aktoreinrichtung ausgelegt ist ein Akustik-Reizmuster und/oder ein Vibrations-Reizmuster zu erzeugen.

9. Vorrichtung zur Beeinflussung des Schlafs einer Person nach einem der vorhergehenden Ansprüche, wobei die erste Sensoreinrichtung einen optischen Sensor aufweist, der auf der Basis des Sagnac-Effektes arbeitet, indem eine Faserkreiseleinrichtung bereitgestellt ist, mit welcher Drehungen des Körpers um die Körperlängsachse erfaßbar sind.

10. Vorrichtung zur Beeinflussung des Schlafs einer Person nach einem der vorhergehenden Ansprüche, wobei die zweite Sensoreinrichtung ein Mikrofon aufweist.

11. Vorrichtung zur Beeinflussung des Schlafs einer Person nach Anspruch 10, wobei die Bandbreite des Mikrofons schnarchspezifisch ist und vorzugsweise zwischen 30 und 1600 Hz liegt.

12. Vorrichtung zur Beeinflussung des Schlafs einer Person nach einem der vorhergehenden Ansprüche, wobei die Empfindlichkeit und/oder Frequenzcharakteristik der zweiten Sensoreinrichtung einstellbar ist.

13. Vorrichtung zur Beeinflussung des Schlafs einer Person nach einem der vorhergehenden Ansprüche, wobei die dritte Sensoreinrichtung einen Herzfrequenzsensor und/oder einen Blutsauerstoffsättigungssensor aufweist.

14. Vorrichtung zur Beeinflussung des Schlafs einer Person nach Anspruch 12, wobei die Herzfrequenz und/oder die Blutsauerstoffsättigung, welche anzeigen, dass der Kreislauf der Person gestört ist, einstellbar sind.

15. Vorrichtung zur Beeinflussung des Schlafs einer Person nach einem der vorhergehenden Ansprüche, wobei ein Haltezeitglied vorgesehen ist, das ausgelegt ist, einen Betrieb der Vorrichtung vorgebbar zu unterbrechen.

16. Verfahren zur Beeinflussung des Schlafs einer Person mit:
a) Erfassen einer aktuellen Drehposition des Körpers der Person um die Körperlängsachse;
b) Auswählen mindestens einer erwünschten und/oder mindestens einer verbotenen Drehposition des Körpers der Person um die Körperlängsachse an einer Bedieneinheit;
c) Zuführen von einem vorgebbaren Reizmuster an den Körper;
d) Vergleichen der aktuellen Drehposition und der mindestens einen erwünschten und/oder mindestens einen verbotenen Drehposition und Ausgeben eines entsprechenden Lagesignals in der Bedieneinheit; und
f) Ansteuern der Aktoreinrichtung zum Zuführen von dem vorgebbaren Reizmuster an den Körper durch die Bedieneinheit, wenn das Lagesignal anzeigt, dass die aktuelle Drehposition nicht der mindestens einen erwünschten und/oder mindestens der einen verbotenen Drehposition entspricht.

17. Bedieneinheit zur Durchführung des Verfahrens nach Anspruch 16 mit:
einem ersten Anschluss zum Anschliessen einer an der Person anbringbarer ersten Sensoreinrichtung zum Erfassen einer aktuellen Drehposition des Körpers der Person um die Körperlängsachse;
einer ersten Auswahleinrichtung zum Auswählen mindestens einer erwünschten und/oder mindestens einer verbotenen Drehposition des Körpers der Person um die Körperlängsachse;
einem zweiten Anschluss zum Anschliessen einer Aktoreinrichtung zum Zuführen von einem vorgebbaren Reizmuster an den Körper;
einer Vergleichseinrichtung zum Vergleichen der aktuellen Drehposition und der mindestens einen erwünschten und/oder mindestens einen verbotenen Drehposition und Ausgeben eines entsprechenden Lagesignals; und
einer Steuereinrichtung, welche einrichtbar ist, ansprechend auf das Lagesignal die Aktoreinrichtung zum Zuführen von dem vorgebbaren Reizmuster an den Körper anzusteuern, wenn das Lagesignal anzeigt, dass die aktuelle Drehposition nicht der mindestens einen erwünschten und/oder mindestens der einen verbotenen Drehposition entspricht.

18. Computerprogrammprodukt mit einem maschinenlesbaren Code, wodurch die Durchführung des Verfahrens nach Anspruch 16 auf einer Bedieneinheit nach Anspruch 17 ermöglicht ist.
